(19) **Europäisches Patentamt / European Patent Office / Office européen des brevets**

(11) **EP 1 497 846 B1**

(12) # FASCICULE DE BREVET EUROPEEN

(45) Date de publication et mention
de la délivrance du brevet:
**10.09.2008 Bulletin 2008/37**

(21) Numéro de dépôt: **03715246.9**

(22) Date de dépôt: **24.04.2003**

(51) Int Cl.:
*H01J 37/32* (2006.01)   *H05H 1/24* (2006.01)
*H05H 1/26* (2006.01)

(86) Numéro de dépôt international:
**PCT/IB2003/001675**

(87) Numéro de publication internationale:
**WO 2003/092039 (06.11.2003 Gazette 2003/45)**

(54) **DISPOSITIF POUR LE TRAITEMENT DE SURFACE DE RECIPIENTS PAR PLASMA**

VORRICHTUNG ZUR PLASMABEARBEITUNG DER OBERFLÄCHEN VON BEHÄLTERN

DEVICE FOR TREATING SURFACES OF CONTAINERS WITH PLASMA

(84) Etats contractants désignés:
**AT BE BG CH CY CZ DE DK EE ES FI FR GB GR HU IE IT LI LU MC NL PT RO SE SI SK TR**

(30) Priorité: **24.04.2002 EP 02405335**

(43) Date de publication de la demande:
**19.01.2005 Bulletin 2005/03**

(73) Titulaire: **Apit Corp. S.A.**
**1950 Sion (CH)**

(72) Inventeurs:
- **KOULIK, Pavel**
  **F-67113 Blaesheim (FR)**
- **SAMSONOV, Mickhail**
  **F-67400 Illkirch-Graffenstaden (FR)**
- **CHEREPANOV, Alexander**
  **F-67400 Illkirch-Graffenstaden (FR)**
- **PETROV, Evguenii**
  **F-67400 Illkirch-Graffenstaden (FR)**
- **BORISSOV, Oleg**
  **F-67400 Illkirch-Graffenstaden (FR)**
- **LOHR, Robert**
  **F-67980 Hangenbieten (FR)**
- **VANAUD, Sébastien**
  **F-67400 Illkirch-Graffenstaden (FR)**
- **RUNG, Vladimir**
  **Toronto M2S 1Z3 Ontario Canada (CA)**

(74) Mandataire: **Reuteler, Raymond Werner**
**WILLIAM BLANC & CIE**
**Conseils en Propriété Industrielle SA**
**Avenue du Pailly 25**
**1220 Les Avanchets/Genève (CH)**

(56) Documents cités:
| | |
|---|---|
| EP-A- 0 636 427 | EP-A- 1 073 091 |
| EP-A- 1 096 837 | WO-A-00/27170 |
| WO-A-95/29860 | WO-A-99/46964 |
| US-A- 6 140 773 | |

- **PATENT ABSTRACTS OF JAPAN vol. 2002, no. 08, 5 août 2002 (2002-08-05) & JP 2002 110397 A (SEKISUI CHEM CO LTD), 12 avril 2002 (2002-04-12)**
- **PATENT ABSTRACTS OF JAPAN vol. 2002, no. 07, 3 juillet 2002 (2002-07-03) & JP 2002 094221 A (SEKISUI CHEM CO LTD), 29 mars 2002 (2002-03-29)**
- **PATENT ABSTRACTS OF JAPAN vol. 2002, no. 05, 3 mai 2002 (2002-05-03) & JP 2002 008895 A (MATSUSHITA ELECTRIC WORKS LTD), 11 janvier 2002 (2002-01-11)**
- **PATENT ABSTRACTS OF JAPAN vol. 2002, no. 05, 3 mai 2002 (2002-05-03) & JP 2002 001253 A (MATSUSHITA ELECTRIC WORKS LTD), 8 janvier 2002 (2002-01-08)**

## Description

La présente invention concerne un dispositif pour le traitement de surface par plasma de récipients, par exemple l'imperméabilisation et la stérilisation de bouteilles en matière plastique, à des cadences industrielles.

Le traitement de surface de récipients par plasma est connu et a été industrialisé ou proposé par diverses sociétés, telles que les sociétés SIDEL, TETRAPAK et KRONES. Les dispositifs proposés ou industrialisés par ces sociétés utilisent des plasmas générés par des générateurs micro-ondes ou hautes fréquences (HF) sous vide. Ces dispositifs et procédés sont décrits dans divers articles, par exemple dans:

(1) le projet ACTIS, proposé, développé et industrialisé par SIDEL. SIDEL News, le journal des clients, septembre 2001 p.8,9,

(2) le projet GLASKIN, proposé, développé et industrialisé par TETRAPAK, Tetrapak Business Area Plastics, September 2001,

(3) le projet BEST PET, proposé, développé et industrialisé par KRONES, Krones News, September 2001,

Ces dispositifs ont le désavantage que la génération du plasma se fait dans le vide et, par conséquent, ils nécessitent un équipement incluant des pompes à vide et des tuyauteries hermétiques. Ceci rend les machines chères, peu flexibles, très encombrantes et difficilement intégrables sur les lignes de remplissage de bouteilles PET dans l'industrie des boissons (bière, eaux minérales, boissons carbonatées, laits et produits laitiers).

Dans le cas de l'équipement proposé par la société SIDEL, où la bouteille est traitée sur une machine du type carrousel, la machine doit être équipée de joints de friction qu'il est difficile de rendre fiables et qui assurent difficilement un vide strictement reproductible d'une bouteille à l'autre.

En plus, le cycle de traitement du container nécessite une étape de mise sous vide, ce qui, en principe, crée une perte de temps dans le processus de traitement.

Des procédés de traitement de surfaces de récipients à plasma atmosphérique, tels que décrits dans la demande de brevet international PCT/IB02/01001 publiée sous le numéro WO 02/076511 A2 permettent d'éviter les inconvénients précités. Cependant, dans les procédés de l'art antérieur ou de ladite demande internationale, un traitement par plasma efficace nécessite une durée de traitement qui est bien supérieure au temps nécessaire pour maintenir une cadence industrielle d'une chaîne de remplissage des récipients industriels.

Par exemple, dans le cas de l'imperméabilisation de bouteilles PET par plasma atmosphérique selon le procédé décrit dans la demande internationale PCT/IB02/01001 publiée sous le numéro WO 02/076511 A2, la durée du traitement est de l'ordre de 30 secondes alors que la ligne de fabrication et de remplissage des bouteilles a une productivité allant jusqu'à 40 000 bouteilles/heure, ce qui correspond à peu près à 10 bouteilles à la seconde. Pour satisfaire à l'exigence de la productivité industrielle, il faut donc, dans ce cas traiter 300 bouteilles simultanément pour assurer la cadence industrielle de 0,1 sec/bouteille. Les dispositifs de traitement, pour l'application industrielle, doivent donc prévoir une accumulation des containers, un traitement des containers en parallèle, et une distribution des containers après leur traitement. La quantité de containers traités en parallèle est égale au produit de la cadence industrielle (productivité) par la durée de traitement d'un container. Par exemple, si la cadence industrielle est 10 bouteilles par seconde et la durée du traitement 30 secondes, la quantité de containers traités en parallèle doit être 300.

Le document WO 99/46964 A décrit un dispositif pour le traitement de surface de récipients par plasma comprenant un générateur de plasma travaillant à pression atmosphérique et apte à générer une décharge du type réseau de filaments, destiné à traiter un récipient à la fois, le générateur de plasma comprenant un système d'alimentation de gaz de traitement, un système d'alimentation en courant électrique et une unité de contrôle.

Au vu de ce qui précède, un but de l'invention est de fournir un dispositif de traitement de surface de récipients, tels que des bouteilles, par plasma atmosphérique, à des cadences industrielles.

Il est avantageux de fournir un dispositif pour le traitement de surface de bouteilles qui peut facilement être intégré dans une chaîne industrielle de fabrication et de remplissage de bouteilles et qui est compact, fiable et économe.

Il est avantageux de fournir un dispositif pour le traitement de surface par plasma comprenant un générateur de plasma travaillant à pression atmosphérique et générant un plasma permettant le traitement de surface de l'intérieur d'une bouteille de haute qualité, notamment la stérilisation et le dépôt de films barrières, par exemple pour des bouteilles PET.

Les buts de l'invention sont réalisés par un dispositif pour le traitement de surface de récipients par plasma atmosphérique selon la revendication 1.

Dans la présente invention, un dispositif pour le traitement de surface de récipients par plasma comprend un système cinématique pour le transport des récipients et une pluralité de générateurs de plasma travaillant à pression atmosphérique, chaque générateur de plasma étant destiné à traiter un récipient à la fois et comprenant un système d'alimentation en gaz de traitement, un système d'alimentation en courant électrique comprenant un bloc de distribution de courant électrique et au moins un transistor agissant comme interrupteur, ou un adaptateur LC, agencé pour alimenter le courant en impulsions et une unité de contrôle montée dans le corps du générateur. Chaque générateur est sous forme d'une colonne d'un diamètre ou d'une largeur proche ou légèrement supérieur au diamètre ou à la largeur du récipient, et apte à générer une décharge du type réseau de filaments.

Dans le système d'alimentation des générateurs comprenant des adaptateurs LC, les impulsions de courant sont générées par une alimentation centrale (collective) et distribuées par des lignes conducteurs, par exemple coaxiales, en parallèle à une pluralité de générateurs de plasma. Les adaptateurs LC de chaque générateur permettent d'accorder la puissance absorbée par la décharge (plasma) à celle générée par l'alimentation centrale, c'est-à-dire d'accorder l'impédance de la charge à celle de la source.

Dans le système d'alimentation des générateurs comprenant des interrupteurs-transistors, les impulsions de courant pour la décharge sont générées ou contrôlées individuellement dans chaque générateur, ces générateurs pouvant donc être connectés à un réseau électrique ou autre source d'énergie électrique externe sans nécessiter les mesures spéciales requises en utilisant une alimentation centrale distribuée en parallèle aux générateurs.

Le système d'alimentation en courant des générateurs peut comprendre, ou être relié à une unité de contrôle agencée pour contrôler l'amplitude et le taux de croissance du front de croissance des impulsions de courant électrique, leur durée et leur fréquence. La durée réglable et la cadence de répétition des impulsions sont donc réglées et contrôlées avec un générateur à transistor de petite dimension et de faible prix. Dans la variante adaptateur LC, les adaptateurs sont aussi de petites dimensions et de faible prix. Ceci permet de munir le dispositif de traitement avec une pluralité de générateurs de plasma afin d'effectuer le traitement en parallèle d'une pluralité de récipients, chacun étant alimenté par un générateur individuel.

L'utilisation de générateurs selon l'invention permet de générer des plasmas à pression atmosphérique, qui sont hors d'équilibre thermodynamique et chimique, le réglage et le contrôle du caractère des impulsions et de leur cadence, notamment du temps entre deux impulsions, permettant de varier l'activité chimique des particules, atomes, molécules, radicaux, clusters excités du plasma, afin d'obtenir la qualité voulue du traitement.

Les générateurs de courant électrique en impulsions, de petites dimensions, peuvent fonctionner en régime forcé, c.à.d les éléments du générateur sont forcés de générer à un régime énergétique plus intense que leur capacité, mais en régime thermique non-stationnaire. En régime forcé, un refroidissement des éléments du générateur entre les impulsions peut être nécessaire et, à cette fin, les impulsions peuvent être séparées par des pauses de durée relativement longue assurant le refroidissement avant l'impulsion suivante. Le régime de transfert de chaleur de ces générateurs en impulsion est donc un régime non-stationnaire.

Les différentes formes d'exécution des systèmes cinématiques selon l'invention, qui seront décrites ci-après en relation avec les figures, permettent d'assurer un transport rapide et un positionnement précis et fiable des récipients sous les générateurs de plasma correspondants.

D'autres buts et caractéristiques avantageux de l'invention, ressortissant des revendications, de la description des formes d'exécution de l'invention ci-après, et des dessins, dans lesquels :

La Fig. 1 représente, de manière simplifiée, une vue de dessus d'un dispositif de traitement de surface de récipients, notamment de bouteilles, par plasma atmosphérique selon l'invention, illustrant en particulier le système cinématique;

La Fig. 2 montre une vue simplifiée de dessus d'une autre forme d'exécution d'un dispositif de traitement de surface par plasma atmosphérique de bouteilles selon l'invention, illustrant en particulier le système cinématique;

La Fig. 3 montre une vue simplifiée en coupe à travers les lignes III-III de la Fig. 2 d'un dispositif de traitement de surface par plasma atmosphérique de bouteilles selon l'invention;

La Fig. 4 montre une vue simplifiée de dessus d'une autre forme d'exécution d'un dispositif de traitement de surface par plasma atmosphérique selon l'invention, illustrant en particulier le système cinématique;

La Fig. 5a montre une vue simplifiée de dessus d'une autre forme d'exécution d'un dispositif de traitement de surface par plasma atmosphérique selon l'invention, illustrant en particulier le système cinématique;

La Fig. 5b montre une vue de côté d'une partie du dispositif de la Fig. 5a;

La Fig. 6 montre une vue simplifiée de dessus d'une autre forme d'exécution d'un dispositif de traitement de surface par plasma atmosphérique selon l'invention, illustrant en particulier le système cinématique;

La Fig. 7a montre une vue simplifiée en perspective d'une autre forme d'exécution d'un dispositif de traitement de surface par plasma atmosphérique selon l'invention;

La Fig. 7b est une vue en coupe simplifiée d'une partie d'un générateur de plasma et d'une bouteille lors du traitement;

La Fig. 8 est une vue simplifiée d'une forme d'exécution d'un générateur de plasma et d'une bouteille lors du traitement selon l'invention;

La Fig. 9 est une vue illustrant une représentation fonctionnelle d'une autre forme d'exécution d'un générateur à transistor de plasma du dispositif selon l'invention;

La Fig. 10 est une vue simplifiée illustrant l'alimentation électrique d'un générateur à transistor selon une variante de l'invention;

La Fig. 11 est un schéma électrique d'une alimentation électrique avec un séparateur optique à haute tension d'un générateur selon une variante de l'invention;

La Fig. 12 est un schéma électrique d'une alimentation électrique basée sur l'utilisation d'un transistor de champ, d'un générateur selon une variante de l'invention;

La Fig. 13 est une vue schématique d'une forme d'exécution d'un dispositif selon l'invention avec une alimentation électrique distribuée à une pluralité de générateurs de plasma à partir d'un bloc d'alimentation centrale;

Les Figures 14a à 14e sont des schémas électriques de différentes variantes d'une alimentation électrique de générateurs de plasma avec adaptateur LC dans le cas où une pluralité de générateurs sont connectés à un bloc d'alimentation centrale tel qu'illustré dans la Fig. 13.

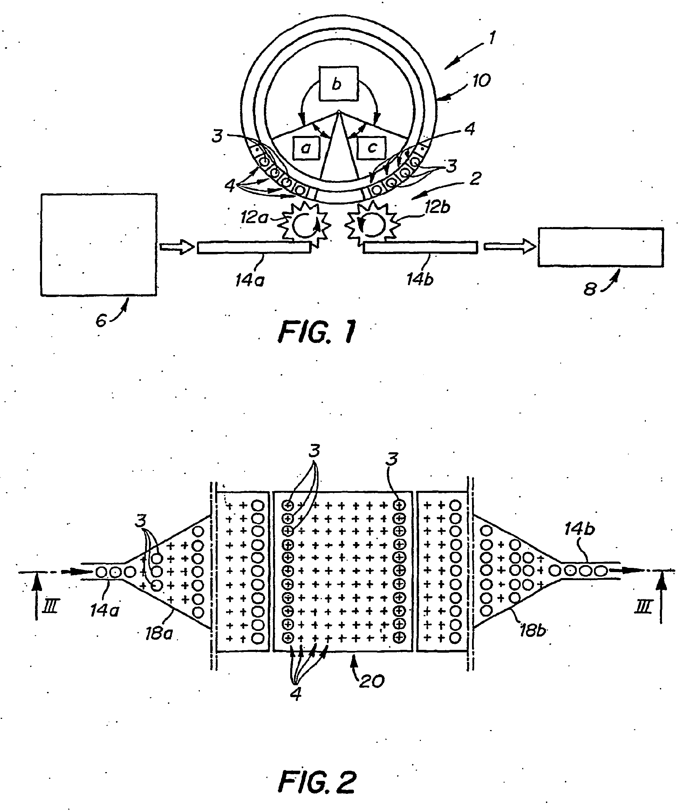

En faisant référence aux figures, notamment les Figures 1 à 8, un dispositif de traitement de surface par plasma atmosphérique de récipients, par exemple pour les traitements tels que la stérilisation et le dépôt de films barrières, de surfaces intérieures de bouteilles PET, comprend un système cinématique 2 pour le transport et le positionnement de récipients lors du traitement, et une pluralité de générateurs de plasma 4. Chaque générateur de plasma 4 est destiné à effectuer un cycle de traitement complet sur un seul récipient à la fois (par exemple nettoyage, activation, dépôt de films et stérilisation), la pluralité de générateurs disposés dans ou le long du système cinématique permettant de traiter simultanément une pluralité de récipients. Chaque générateur comprend un système d'alimentation en gaz de traitement et un système d'alimentation de courant par la génération des décharges (plasma).

Dans le dispositif selon l'invention, autant de générateurs de plasma que de nombre de bouteilles traitées en parallèle sont donc disposés afin de pouvoir maintenir la cadence industrielle de fabrication et de remplissage des récipients dans une chaîne de production industrielle. Par exemple, si un cycle de traitement de surface d'une bouteille par plasma atmosphérique dure 30 secondes et la cadence de la chaîne de fabrication industrielle est de 10 bouteilles par seconde, on devrait effectuer un traitement par plasma sur 300 bouteilles en parallèle. A cet effet, afin de répondre à différentes cadences industrielles, les dispositifs de traitement par plasma peuvent par exemple traiter simultanément entre 10 et 100 bouteilles, plusieurs dispositifs pouvant être mis en parallèle pour augmenter le nombre de traitements simultanés. D'autre part, l'utilisation de plusieurs dispositifs de traitement permet d'assurer la continuité de production au cas où une défaillance dans un dispositif interviendrait et/ou pour effectuer des opérations de maintenance.

Un des avantages important du dispositif selon l'invention est que les générateurs de plasma à pression atmosphérique selon l'invention sont de petites dimensions et d'une construction relativement simple leur permettant d'être disposés dans un système cinématique relativement compact pour le traitement d'un grand nombre de récipients en parallèle. D'autre part, dans une forme d'exécution préférée, les générateurs sont agencés de manière à créer des plasmas (hautes fréquences ou unipolaires) par impulsions avec un front de croissance de l'impulsion très rapide pour satisfaire par exemple aux conditions données dans la demande internationale PCT/IB02/01001. Les conditions précitées permettent d'assurer un très bon traitement de surface des récipients à pression atmosphérique, évitant entre autre les problèmes liés au traitement de plasma sous vide partiel.

Comme il sera décrit plus en détail ci-dessous en relation avec les différentes formes d'exécution, les deux types principaux de générateurs, qui peuvent être miniaturisés et sont aptes à générer les impulsions de plasma voulues, sont soit des générateurs avec des alimentations individuelles contrôlées par des unités de contrôle disposées dans chaque générateur et comprenant un interrupteur-transistor pour la création des impulsions, soit des générateurs de plasma alimentés en parallèle par un seul bloc d'alimentation, chaque générateur comprenant un adaptateur LC pour accorder l'impédance de la décharge à celle de la source de courant haute fréquence, haute tension.

Les générateurs de plasma peuvent être disposés et utilisés en relation avec différents systèmes cinématiques qui seront maintenant décrits en relation avec les figures 1 à 8.

Faisant référence à la Fig. 1, le dispositif 1 de traitement de récipients 3 comprend un système cinématique 2 selon une première forme d'exécution, disposé entre une station de fabrication ou de chargement 6 de bouteilles et une station de remplissage 8 des bouteilles. Le système cinématique 2 comprend un carrousel 10 sur lequel sont montés une pluralité de générateurs de plasma 4, sous laquelle les récipients 3 sont chargés et déchargés par des roues étoiles 12a, 12b à partir, respectivement sur, des convoyeurs 14a, 14b. Dans le dispositif selon la Fig. 1, le traitement du récipient s'effectue donc lors du déplacement du récipient (par exemple de la bouteille) sur le carrousel. Le récipient vient soit d'une palette soit d'une souffleuse, par exemple de bouteilles 3 en PET ou en verre, par le convoyeur 14a et la roue étoile 12a. Dès qu'il est fixé sur le carrousel, sous les générateurs de plasma 4, le traitement a lieu. On distingue trois secteurs de différents traitements : le secteur (a) où le récipient est purgé de l'air qu'il contient par un jet d'argon ou d'azote, le secteur (b), où le récipient est traité (dépôt de barrières), le secteur (c) où le récipient est purgé des gaz résiduels par un jet d'air. Le récipient sort du carrousel au moyen de la roue-étoile 12b et le convoyeur 14b l'emmène à la station de remplissage 8. Comme l'on effectue non seulement un traitement de dépôt de barrière, mais simultanément un traitement stérilisant, la longueur du parcours jusqu'à la station de remplissage 8 doit être minimisée de manière à réduire la recontamination du récipient. Des mesures de manipulation aseptiques conventionnelles peuvent être prises pour éviter la re-contamination du récipient.

Le dispositif peut également comprendre un système de ventilation 15 qui sert à évacuer les gaz résiduels et à refroidir le récipient.

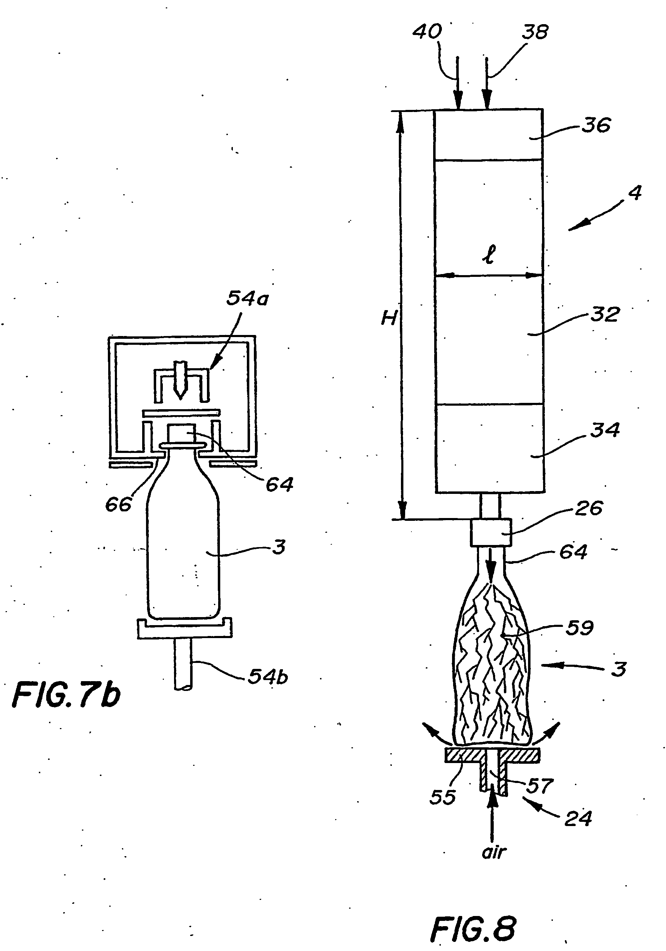

Les générateurs de plasma comprennent chacun un système d'alimentation en gaz et un système d'alimentation en électricité comprenant, ou reliés à, une unité de contrôle du processus qui peuvent être construits selon l'une des formes d'exécution décrites en relation avec les figures 8 à 14e. Chaque générateur peut aussi comprendre ou être associé à un mécanisme de rotation du récipient.

Le traitement des récipients dans le cas de la fig. 1, s'effectue lors du mouvement du récipient emporté par le carrousel. Dans ce cas, les apports de gaz se font par l'intermédiaire de joints de friction et l'apport d'électricité par l'intermédiaire d'un contact électrique de friction (non montrés sur la fig. 1). Le dispositif de rotation du récipient peut toutefois être absent si des mesures sont prises pour assurer le traitement uniforme de toute la surface du récipient, tel que proposé dans certaines formes d'exécution décrites dans la demande internationale PCT/IB02/01001.

La Fig. 2 illustre un autre mode de réalisation du dispositif de traitement par plasma 1 où le traitement s'effectue sur un groupe de récipients en "Batch". Dans ce cas, les récipients 3 sont traités à l'arrêt. Il n'y a pas besoin de joints de friction et de contacts électriques de friction. Les récipients venant du convoyeur 14a s'accumulent dans un sas d'accumulation 18 et sont transmis dans une zone de traitement 20 où ils sont traités simultanément par une pluralité de générateurs de plasma 4, une par bouteille dans la zone de traitement. Ensuite, les récipients passent dans le sas de distribution 22 et repartent sur le convoyeur. Ce schéma est avantageux car il ne nécessite pas d'éléments de contact électrique par friction.

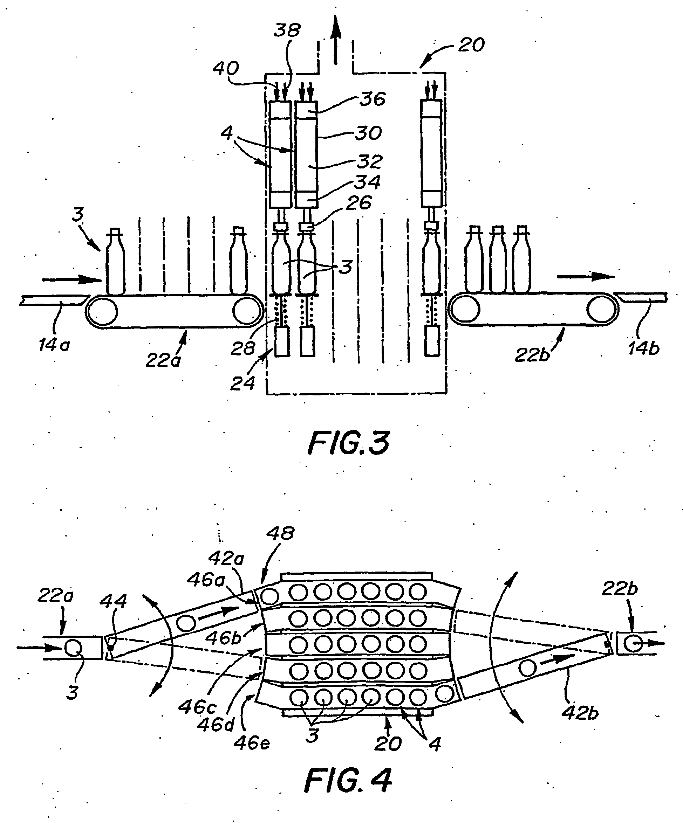

La Fig. 3 montre une section du dispositif de la Fig. 2. Les récipients, par exemple des bouteilles PET, viennent de la ligne de fabrication par le convoyeur 14a. Ils sont distribués dans le sas d'accumulation 18 (non montré sur la Fig. 3) et accèdent à l'aide d'un transporteur 22a à la zone de traitement 20 où ils sont fixés sur un mécanisme de rotation 24 sous un des générateurs de plasma 4 comprenant un capuchon 26 mis en contact avec le goulot du récipient, l'effort de contact étant exercé par exemple par un ressort 28. Dans le capuchon est apporté le gaz de traitement, par exemple un mélange gazeux, par l'intermédiaire d'une électrode tubulaire (non montré sur la Fig. 3, mais décrit plus en détail en rapport avec les figures 8 à 14e). L'électrode assure le passage du courant dans le récipient à traiter à partir d'un système d'alimentation de courant 30 disposé dans le corps 32 du générateur. Outre la source de courant 30, le générateur comporte un système d'alimentation de gaz sous forme d'un distributeur de gaz 34, principalement constitué de tubes, d'électrovannes et d'un vaporisateur (non montrés sur la fig.3). La source de courant et le distributeur de gaz sont contrôlés par une unité de contrôle ou un microcontrôleur 36, également monté dans le corps du générateur. Des entrées de gaz 38 et d'électricité 40 sont prévues à la partie supérieure du générateur. Le mécanisme de rotation 24 peut comporter un moteur électrique pour assurer la rotation du récipient pendant son traitement.

Un système de ventilation des récipients peut aussi être disposé dans le dispositif pour assurer l'évacuation des gaz résiduels et le refroidissement des récipients pendant leur traitement.

Après le traitement, les récipients sont repris par un transporteur 22b et remis, par l'intermédiaire du sas d'évacuation 18b (voir Fig. 2), sur le convoyeur 14b vers la station de remplissage.

La Fig. 4 illustre un mode de réalisation de la présente invention consistant à charger la zone de traitement 20 en récipients 3 au fur et à mesure de leur traitement, à l'aide du mécanisme de chargement 42a du système cinématique, comprenant un guide pivotant autour de l'axe 44.

Dans ce cas, le traitement s'effectue par rangs 46a à 46b. Dès qu'un rang du système cinématique a été chargé de récipients 3, le traitement de surface par plasma y est amorcé. Pendant que le traitement s'effectue dans les récipients de ce rang, les autres rangs sont chargés. Dès qu'un rang est traité, il est déchargé par un mécanisme de décharge 42b analogue à celui de chargement. Deux zones de transition 48 permettent un chargement précis et sans heurts. Ce dispositif a l'avantage par rapport à celui de la Fig. 3, de ne pas contenir de sas d'accumulation et de déchargement des récipients. Il est donc de faible encombrement par rapport aux systèmes des Figures 1 et 2.

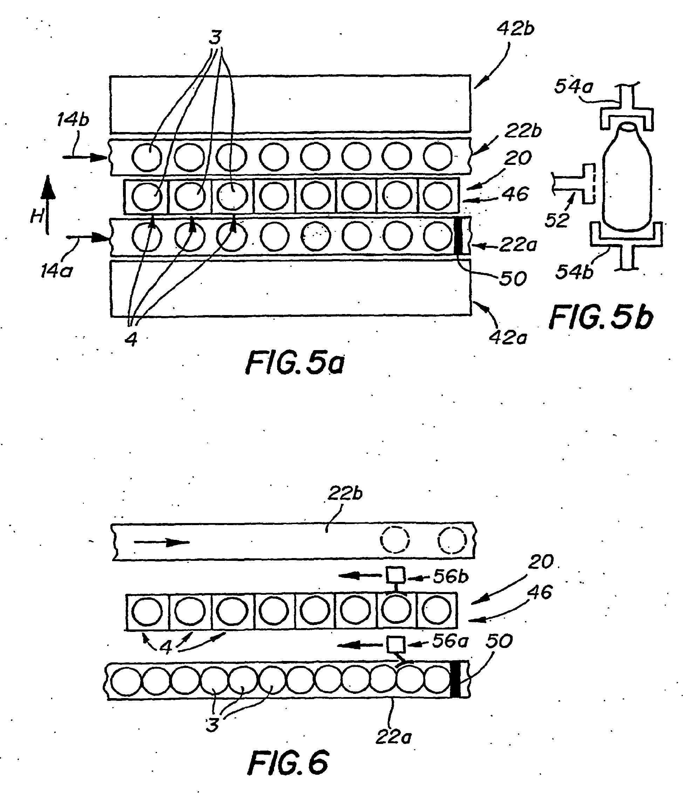

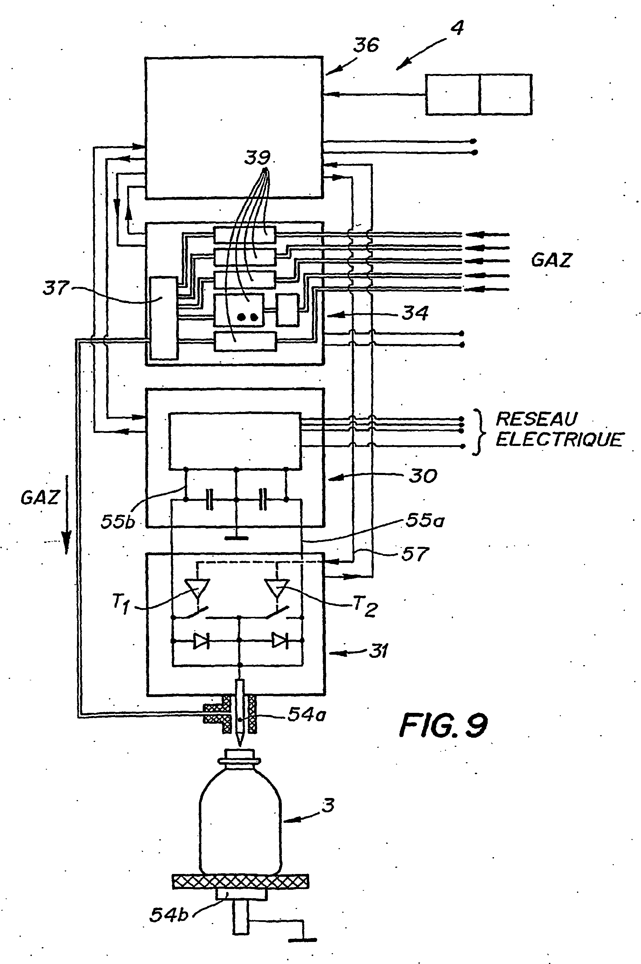

La Fig. 5 montre un dispositif de traitement de surface par plasma de récipients selon l'invention, comprenant un système cinématique avec chargement et déchargement simultanés des bouteilles en ligne (rang). Dans ce cas, on peut utiliser des générateurs de plasma tels que représentés sur les Figures 8 et 9. Des systèmes d'alimentation en eau (ou autre agent de refroidissement), gaz de traitement et électricité se situent au-dessus et en-dessous du rang de traitement 46. En parallèle avec ce rang 46 sont montés deux transporteurs 22a et 22b à côté desquels sont situés les dispositifs 42a et 42b de chargement et déchargement qui assurent le placement simultané des bouteilles 3 venant du convoyeur 14a dans la zone de traitement 20 et leur déchargement de la zone de traitement vers le convoyeur de déchargement 22b.

La machine fonctionne comme suit : les bouteilles arrivent par le convoyeur 14a l'une contre l'autre. Elles sont arrêtées par un buttoir 50, sont séparées les unes des autres par un système pneumatique 52 et sont transportées ensemble dans une direction horizontale H dans la zone de traitement 20 formée par le rang 46. Ici, les bouteilles sont en prise du dessous et du dessus entre les électrodes 54a et 54b. L'aspireur 52 libère les récipients et revient en position initiale 42a. Le traitement de surface par plasma de l'intérieur des récipients commence. Pendant ce temps, sur le convoyeur d'entrée, se répètent les opérations décrites de manière à ce que, au moment voulu (sans perte de temps) la bouteille suivante puisse être placée dans la zone de traitement.

La libération de la zone de traitement des bouteilles traitées se fait après la fin du traitement. A ce moment, du côté du mécanisme de déchargement vient un aspireur et les électrodes 54a, 54b libèrent les bouteilles et celles-ci sont déplacées par l'aspireur vers le mécanisme de déchargement 22b. Les aspireurs libèrent les bouteilles et celles-ci quittent le dispositif de traitement vers la station de remplissage.

Par exemple, si l'on veut traiter K=10 800 bouteilles/heure soit 3 bouteilles/sec et T, le temps de traitement est par exemple T=30sec;

T le temps de remplacement des bouteilles (chargement + déchargement) est par exemple 3 sec;

d le diamètre de la bouteille, est par exemple d=0,06m; et I la longueur de l'espace nécessaire pour une bouteille est par exemple I=0,1 m/bouteille.

Le nombre d'espaces dans la machine est : $N= (T+ _T).K$ dans notre exemple :

$$N= (30+3).3 \approx 200$$

La longueur de la machine est : L=N.I dans notre exemple : L=100.0,1=10m

La largeur de la machine est :

$$B=2a+b+2c$$

Où a est la largeur des zones 4 et 5 (~ 0,3m) b est la largeur de la zone 1 c est la largeur du convoyeur (~ 0,1 m)

Dans notre exemple B ~ 0,9m

La vitesse de mouvement des bouteilles le long du convoyeur doit être non moins de : $W=N.d/T+ _T=K.d$ Dans notre exemple W=0,18m/sec.

Le temps de séparation des bouteilles sur le convoyeur d'arrivée ne détermine pas la productivité de la machine, car il fait partie du temps de traitement. Cependant, sa grandeur doit correspondre à l'inégalité : $td \leq T-L/W$

En cas de nécessité on peut varier la vitesse W. L'avantage de cette configuration est que la machine a de petites dimensions et peut être installée le long d'une ligne de convoyage de bouteilles.

Un inconvénient du dispositif précité est que les mécanismes de séparation et de déplacement des bouteilles sont volumineux ($\geq$10m). En outre, les bouteilles PET sont très légères et lors de leur mouvement transversal, elles peuvent perdre leur équilibre.

La Fig. 6 illustre le schéma de réalisation d'une machine linéaire avec chargement et déchargement des bouteilles 3 en série.

Les générateurs de plasma sont disposés en ligne (rang 46) et de chaque côté de cette ligne sont situés des convoyeurs de chargement et de déchargement 22a, 22b. Le placement des bouteilles dans la zone de traitement 20 définie par le rang 46 et leur libération de cette zone se font en série et individuellement par des robots de manutention 56.

La machine fonctionne comme suit : les bouteilles 3 arrivent continuellement par le convoyeur de chargement 22a et s'y accumulent, l'une contre l'autre, après avoir butté contre le buttoir 50. Le robot de chargement 56a se déplace parallèlement au rang 46 dans un sens par exemple de droite à gauche, prend avec son aspireur une bouteille du convoyeur et la place dans l'emplacement de traitement le plus proche (la place libérée du convoyeur de chargement 22a se remplit immédiatement par une autre bouteille). Ensuite, le robot se retourne de 180° et place la bouteille exactement dans la zone de traitement. Des électrodes supérieure et inférieure emprisonnent la bouteille, l'aspireur se détache et le robot se déplace pour effectuer la même manipulation de manière à remplir la zone suivante de traitement sous un générateur de plasma 4. Arrivé à la fin du rang 46, dans un sens, le robot revient rapidement en position initiale et recommence son travail.

Le déchargement des bouteilles se fait symétriquement par le robot 56b, qui prend avec son aspireur les bouteilles traitées et les met sur le convoyeur de déchargement 22b.

Arrivé à la fin de la ligne, le robot revient rapidement en position de départ. Les bouteilles sont évacuées par un convoyeur rapide du système cinématique, par exemple un aéroconvoyeur.

Pour que ce système fonctionne, il faut satisfaire à la condition suivante pour la vitesse du convoyeur de déchargement : $W.\Delta t \geq 2d-I$, où At est le temps entre le déchargement de deux zones de traitement voisines.

Dans ce cas, la largeur totale de la ligne ne varie pas. Il est avantageux d'utiliser des robots compacts avec beaucoup de degrés de liberté et fonctionnant avec beaucoup de précision. Les avantages de cette forme d'exécution sont qu'on n'a pas besoin de système de séparation des bouteilles. En outre, les dispositifs de chargement et déchargement occupent peu de place.

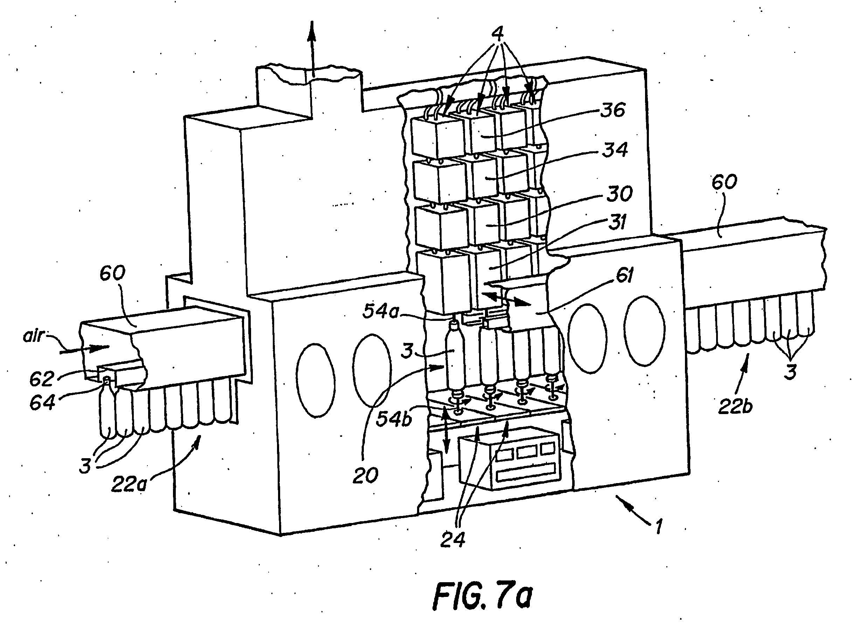

Les Figures 7a et 7b illustrent une autre forme d'exécution d'un dispositif selon l'invention, comprenant un système cinématique ayant un convoyeur à air pour bouteilles. Dans ce cas la ligne de traitement coïncide avec le convoyeur à air de la chaîne de fabrication et de remplissage des bouteilles. Le dispositif est donc monté sur le convoyeur à air d'une chaîne de fabrication et de remplissage des bouteilles sur lequel s'effectue l'opération de traitement de surface par plasma dans une zone de traitement 20 et le chargement et le déchargement des bouteilles dans des parties annexes 22a, 22b. La partie principale de la zone de traitement par plasma comprend des électrodes 54a, 54b, un générateur de plasma 4 installé au-dessus de chaque bouteille 3 et des mécanismes de déplacement et de positionnement des bouteilles dans la zone de traitement.

Le dispositif fonctionne de la manière suivante :

Comme pour un convoyeur habituel à air, les bouteilles sont poussées par de l'air comprimé provenant d'une tubulure 60 et soufflées dans un canal de transport à air 62 agissant comme rail de support pour les goulots 64 des bouteilles. Un compteur de bouteilles laisse entrer le nombre nécessaire de bouteilles, égal à la quantité de postes de traitement, c'est-à-dire de générateurs de plasma 4. Un buttoir les arrête à un endroit précis et un mécanisme de séparation et positionnement des bouteilles les positionne sous les générateurs de plasma 4 respectifs. Il y a plusieurs possibilités de séparation : par exemple au moyen d'une vis d'Archimède, ou d'un peigne à dents coniques, ou d'un système de buttoirs activés les uns après autres au signal de photo-diodes, prévenant de la position des bouteilles. Le mécanisme de positionnement dispose les bouteilles précisément dans l'axe des électrodes 54a, 54b des générateurs de plasma. Les parois 61 formant le la tubulure d'air dans la zone de traitement 20 se déplacent et ouvrent l'accès aux bouteilles 3. L'électrode supérieure 54a descend et l'électrode inférieure 54b monte. L'électrode inférieure 54b est pourvue d'un mécanisme de rotation de la bouteille par l'intermédiaire de patins de frottement. Le goulot 64 de la bouteille glisse, lors de cette rotation, sur la partie intérieure 66 du boîtier de l'électrode supérieure. Un ressort monté sur l'électrode inférieure (non montrée) exerce la pression nécessaire pour assurer, d'une part l'absence de friction entre la bouteille et l'électrode inférieure, et, d'autre part, la friction entre le boîtier (qui est par exemple en téflon) de l'électrode supérieure et le goulot de la bouteille. Le traitement de surface par plasma commence avec le remplissage de la bouteille, par le mélange gazeux formant le gaz de traitement.

Pendant ce temps les deux moitiés amovibles de la tubulure d'air 61 s'écartent de manière à éviter toute interférence et toute influence sur la répartition des lignes de champ électrique pendant le traitement de la bouteille. Les électrodes sont mises sous tension et le procédé de dépôt de film commence. Après un temps T, le procédé s'arrête et toutes les opérations décrites ci-dessus s'effectuent inversement. La bouteille est purgée par un flux d'air, dégagée des électrodes et emportée par le flux d'air.

Les avantages de cette forme d'exécution sont :

- ses petites dimensions
- sa simplicité
- la grande vitesse d'entrée et d'évacuation des bouteilles
- l'impossibilité de tombée des bouteilles
- l'universalité de la machine par rapport au traitement de bouteilles de différents calibres et formes.

La longueur de la machine L dépend de sa cadence de production N, de la durée du cycle de traitement T d'une bouteille et de la largeur I du générateur de plasma.

Pour N=10 000 bouteilles/heure, T=30sec, I=0,1m, nous obtenons L=10m. La dimension transversale de la machine B dépendra du nombre de rangs de générateurs formant la zone de traitement et pour un à deux rangs elle peut être de B=0,5m. Les durées de chargement et de l'évacuation des bouteilles sont déterminées par la vitesse moyenne du mouvement de la bouteille, qui est par exemple de l'ordre de 10m/sec. La durée totale du chargement et du déchargement ne dépasse donc pas $_T$=2sec.

Le générateur de plasma 4 a la forme générale d'une colonne, en un seul bloc, tel que montré dans la Fig. 8, ou en plusieurs blocs tels que montrés dans les figures 7a ou 9, la largeur du générateur étant proche du diamètre de la bouteille ou de la largeur I du récipient, ou légèrement supérieure afin de pouvoir disposer la pluralité des générateurs le long de la chaîne cinématique dans la zone de traitement, de manière compacte. A titre d'exemple, en cas de traitement de surface de bouteilles PET de 0,7 I ayant la forme illustrée dans la Fig. 8, le générateur selon l'invention peut être construit avec une largeur I d'environ 80 mm et une hauteur H d'environ 500 mm. Dans l'exemple donné dans la Fig. 8, le récipient 3 est supporté sur un support rotatif inférieur 55 du mécanisme de rotation 24 muni d'un conduit d'air 57 qui permet de fixer la position de la bouteille et d'empêcher son renversement grâce à l'effet de Bernouilli. Dans cette variante, le générateur sous forme de colonne comprend les éléments déjà décrits en relation avec la Fig. 3 et dont les mêmes numéros de référence sont utilisés. La Fig. 8 illustre également la décharge qui est générée sous forme d'un réseau ramifié de filaments 59, comme décrit dans la demande PCT/IB02/01001.

Faisant référence à la Fig. 7a, le générateur comprend une pluralité de blocs comprenant un bloc de distribution des impulsions de courant électrique (système d'alimentation de courant) 30, un bloc de distribution de gaz (système d'alimentation de gaz) 34 et une unité de contrôle 36.

Dans le bloc de distribution du courant électrique 30 illustré sur la Fig. 7, le courant alternatif du réseau (380V/220V, 50Hz) est d'abord redressé par un redresseur 33 en courant continu à haute tension de pôles positif et négatif (par exemple +20kV et -20kV). Ensuite le courant est transformé en impulsions par un interrupteur-transistor à haute fréquence 31.

Le bloc de distribution des gaz 34 comprend un collecteur 37 dans lequel pénètrent plusieurs composants gazeux par l'intermédiaire d'électrovalves 39. Au collecteur peuvent aussi accéder des vapeurs organométalliques, entraînées par un gaz porteur tel que de l'argon.

Les blocs cités sont actionnés et contrôlés par l'unité de contrôle 36.

Le dispositif d'alimentation en gaz 34 est miniaturisé et disposé à une distance minimale du récipient à traiter de manière que le laps de temps Δ entre le moment du fonctionnement des électrovalves 39 et le moment du remplissage du récipient à traiter égal au rapport du volume V des espaces (tuyaux, valves) au débit du gaz soit inférieur à la différence entre la durée du premier régime (par exemple, la purge du récipient par de l'argon ou de l'azote) et la durée d'établissement du régime stationnaire (c'est-à-dire quand le débit est stationnaire) de cette opération.

En ce qui concerne les générateurs qui peuvent être utilisés avec les dispositifs, et notamment le système cinématique des dispositifs décrits ci-dessus et qui répond aux critères de faible dimension et de coût raisonnable, trois types sont proposés dans le cadre de cette invention. Il convient de remarquer que les trois types proposés sont capables de générer des décharges électriques selon les critères proposés dans l'invention décrite dans la demande internationale PCT/IB02/01001, qui permettent d'effectuer un traitement par plasma à pression atmosphérique avec des performances et une qualité de traitement de surface très élevée. A cet effet, il s'agit notamment de pouvoir générer des décharges avec des impulsions électriques satisfaisant aux conditions avantageuses exposées dans la demande internationale précitée.

Les trois types de générateurs de plasma utilisables dans la présente invention peuvent être résumés comme suit:

1. Alimentation individuelle des décharges dans chaque récipient à traiter à partir de générateurs individuels à haute fréquence (HF), en utilisant des clés semi-conductrices pour transformer un courant continu en impulsions HF pour créer les décharges électriques.

2. Alimentation individuelle des décharges dans chaque récipient à traiter à partir de générateurs individuels générant du courant à haute fréquence en utilisant des transistors, pour créer les décharges électriques.

3. Alimentation en parallèle des décharges avec des impulsions électriques de paramètres essentiellement identiques à partir d'une source de courant central à haute fréquence et haute puissance alimentant une pluralité de générateurs de plasma générant lesdites décharges.

Les générateurs selon l'invention se distinguent des générateurs conventionnels à haute fréquence qui utilisent des lampes diodes mais qui sont de grande taille et inadaptés à une alimentation individuelle d'une pluralité de bouteilles dans un dispositif d'un coût et d'un encombrement acceptables dans l'industrie. Les types de générateurs proposés dans le cadre de la présente invention permettent non seulement une miniaturisation des générateurs de plasma, mais également de créer des décharges par des impulsions de courant dont le front d'impulsion, la durée d'impulsion et la fréquence des impulsions correspondent aux conditions avantageuses décrites dans la demande PCT/IB02/01001.

Considérons d'abord les générateurs du premier type susmentionné.

Dans les générateurs du premier type, on utilise un transistor à haute fréquence de champ selon un schéma d'excitation extérieure, ce qui assure la stabilité de la fréquence quand la charge (la décharge de plasma) est dynamique.

Dans les schémas connus de générateurs, étant donné les effets non désirés de la capacité parasite du transistor (effets de capacité de Miller), le temps en position de travail est agrandi et, pour cette raison, la fréquence de fonctionnement d'un tel générateur ne dépasse pas 150-200kHz.

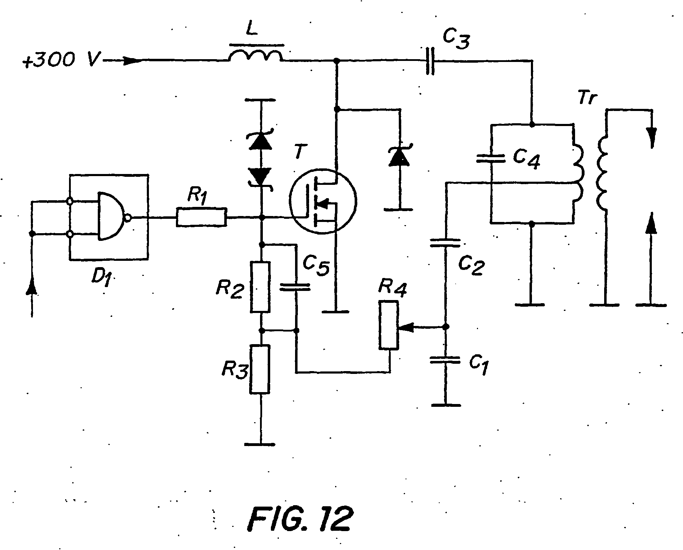

Faisant référence à la Fig. 12, pour obtenir une fréquence entre 1 et 100MHz, la présente invention se distingue des schémas connus en ce que, il est proposé un schéma de générateur sur transistor de champ dans lequel l'influence de la capacité "Miller" est compensée par un circuit $C_1C_2C_5R_2R_3R_4$. L'idée de la compensation consiste en ce qu'une partie de la tension de sortie du transformateur $T_M$, par l'intermédiaire du circuit (amplitude-phase) mentionné, est transmise à la porte (gate) du transistor T et, en s'ajoutant en phase à la tension de contrôle, augmente la deuxième dérivée du courant de rechargement de la capacité de la porte au début et à la fin de l'impulsion d'entrée et conditionne le régime en avalanche du transistor. Le temps de commutation du transistor, de cette manière, est substantiellement réduit, et la fréquence, augmentée, de telle sorte que les exigences du régime du plasma créé selon l'invention décrite dans PCT/IB02/01001, notamment la durée du front des impulsions en dessous de 1 μsec, sont satisfaites.

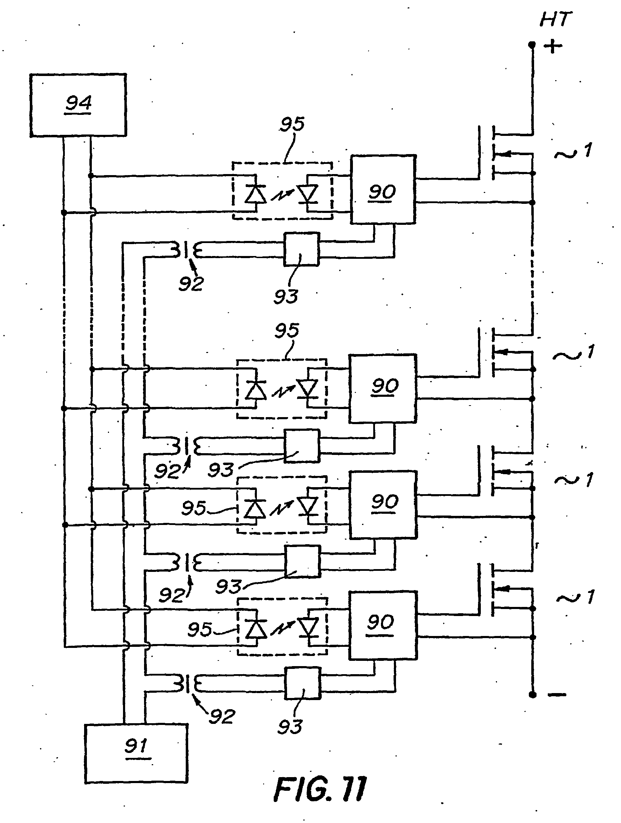

On peut utiliser ce principe quand les transistors de champ sont connectés en parallèle, tel que montré dans la Fig. 11 : dans ce cas leur quantité est déterminée par la puissance de sortie voulue.

Pour assurer le fonctionnement stable du générateur, et éviter des variations de fréquence dues, par exemple, à un échauffement de certains des éléments du générateur, on peut utiliser un système classique de réglage automatique de la fréquence.

Exemple 1.1 :

On a fabriqué un générateur pour la création d'une décharge de plasma atmosphérique sous forme de réseau de filaments dans des bouteilles PET. Pour chaque bouteille, les paramètres du générateur étaient :

Fréquence de génération : 880kHz
Puissance de sortie par impulsion : 6kW
Dimensions : diamètre : 70mm
hauteur : 400mm
Alimentation : 300V (courant continu)

Le circuit utilise 6 transistors en parallèle, du type 2SK2611 et un Driver-TLP250 (du fabricant Toshiba) ($D_1$ sur la Fig. 12).

Les générateurs à haute fréquence selon le deuxième type tel que montré dans la Fig. 9 sur la base de commutateurs-transistors à haute tension et à haute vitesse, se distinguent des générateurs traditionnels par ses petites dimensions, l'absence de diodes et de contours de résonance.

La génération d'une haute tension à haute fréquence s'effectue par la connexion consécutive de l'électrode de la décharge aux pôles positif et négatif d'une source haute tension, par exemple à courant continu.

La fréquence de connexion et la modulation de la tension se contrôlent par ordinateur. On peut obtenir une fréquence entre 1 et 100MHz.

L'interrupteur-transistor (ou commutateur-transistor) 31 se situe directement au-dessus du récipient à traiter, par exemple, de la bouteille PET.

L'interrupteur-transistor est connecté à une source extérieure bipolaire de courant à haute tension et à une unité de contrôle 36 relié à un ordinateur.

En utilisant un schéma de pont basé sur deux transistors $T_1$, $T_2$, on peut utiliser une source extérieure unipolaire de courant à haute tension.

L'interrupteur-transistor à haute vitesse et haute tension 31 est connecté à une électrode 54a par laquelle le courant passe dans le récipient 3 (par exemple une bouteille en PET), par deux bornes de connexion 55a, 55b aux pôles d'une source extérieure bipolaire de courant à haute tension, et par une borne 57 reliée à l'unité de contrôle 36.

Exemple 2.1 :

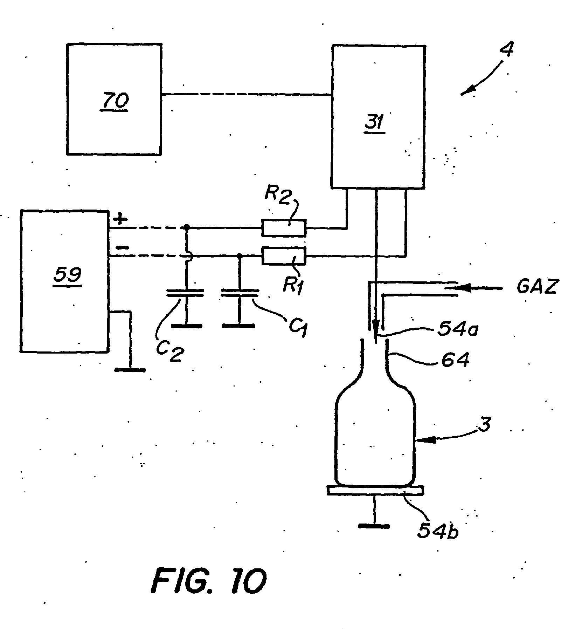

Faisant référence à la Fig. 10, un générateur pour l'alimentation d'une décharge en impulsions en réseau ramifié à l'intérieur d'une bouteille PET a été réalisé en utilisant un interrupteur-transistor 31 à haute tension et haute vitesse du type HTS 301-03-GSM (fabriqué par la société Behlke).

L'interrupteur était alimenté par une source bipolaire 59 de courant à haute tension (-12kV, +12kV, 25kW), par l'intermédiaire d'un circuit RC ($R_1 C_1$, $R_1 C_2$).

L'électrode métallique tubulaire 54a était située à proximité du goulot 64 d'un récipient 3 en plastique. Le gaz générateur contenant des vapeurs d'héxaméthyldisiloxane était introduit par l'électrode. La fréquence de la haute tension alternative et sa modulation étaient déterminées par des impulsions de contrôle venant d'un ordinateur 70. Une décharge sous forme de réseau ramifié a été de cette manière générée le long de la surface intérieure du récipient. Un film imperméable de $SiO_x$ a été créé. Le BIF (barrier Improvement Factor) par rapport à l'oxygène était de 60.

L'interrupteur-transistor 31 utilisé est construit sur la base de transistors de champ à haute tension selon la Fig. 11, connectés en série et alimentée par une source de courant à haute tension. Ce dispositif se distingue des dispositifs conventionnels en ce que l'on utilise un séparateur optoélectronique de haute tension, permettant de transmettre des impulsions avec des fronts de l'ordre de la nanoseconde.

L'interrupteur fonctionne de la manière suivante :

L'enclenchement et le déclenchement des transistors s'effectue à l'aide du driver 90, alimenté par la source 91 ($\sim$ 50kHz) sur les transformateurs 92, dont les enroulements primaires sont en série. Les transformateurs 92, sont faits sur ferrites, et immergés dans de la résine isolante. La tension alternative des enroulements secondaires se redresse par les ponts semi-conducteurs 93 et est transmise aux drivers 90. Le générateur à impulsions 94 crée des paquets d'impulsions envoyés aux drivers par l'intermédiaire des paires optiques à haute tension 95. Il est possible d'envoyer des impulsions lumineuses d'un laser semi-conducteur sur photodiodes à l'aide de fibres optiques.

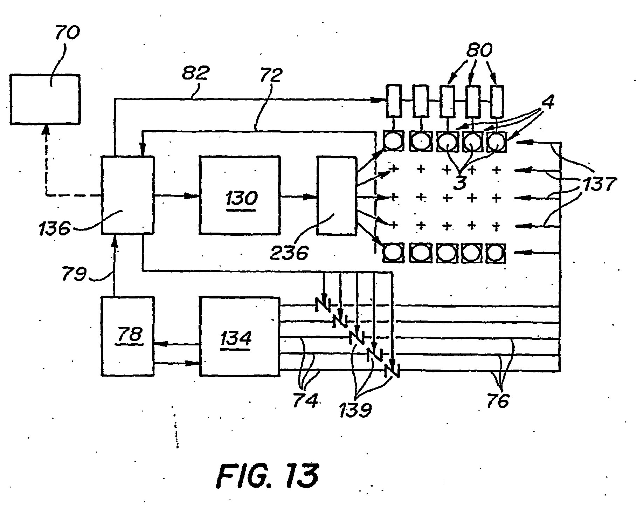

La Fig. 13 est un schéma d'un dispositif avec des générateurs de plasma selon le troisième type mentionnée précédemment, c'est-à-dire où la distribution du courant et le mélange des gaz se font de manière centralisée.

Le cycle du traitement par plasma est contrôlé par l'intermédiaire d'une unité de contrôle central 136 et enregistré sur un ordinateur 70. L'ordinateur fixe le programme (Software) dans un microcontrôleur 236. Ce dernier gère automatiquement le fonctionnement programmé de la source de courant 130 par la ligne 72, du mélangeur de gaz 134, par les lignes 74 avant les électrovalves contrôlées 139 et après 76 du vaporisateur 78 avec retour du signal "température" 79, ainsi que des moteurs de rotation 80 par la ligne 82. Le mélange gazeux a accès à la rampe des générateurs de plasma 4 par l'intermédiaire des collecteurs 137.

Le fonctionnement simultané de plusieurs décharges électriques (jusqu'à 200-300) dont le comportement des paramètres dans le temps est substantiellement non-linéaire, à partir d'un seul générateur, est difficilement réalisable sans mesures spéciales.

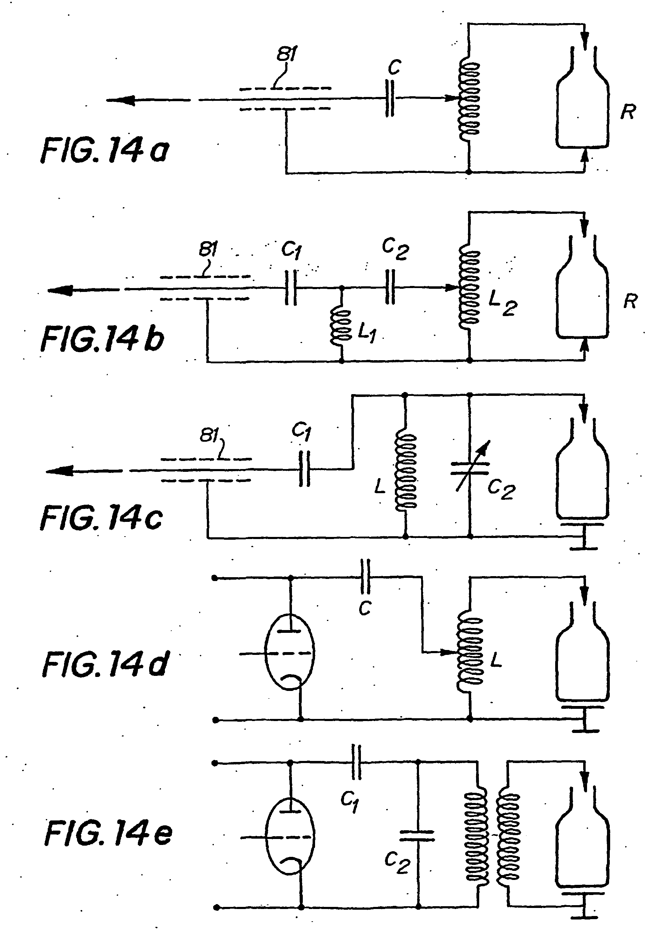

Dans le troisième type de générateur selon la présente invention, on peut utiliser une source de courant haute fréquence standard, de moduler le signal à haute fréquence, c.à.d d'obtenir des impulsions de forme et d'amplitude données dans le temps, en utilisant la méthode standard consistant à envoyer les signaux correspondants à la grille d'une triode. Selon l'invention, on alimente les décharges dans les récipients par l'intermédiaire d'adaptateurs LC individuels tels qu'illustrés dans les figures 14a à 14e, disposés dans le générateur de plasma directement au-dessus du récipient à traiter, à une distance telle que les pertes inductives et capacitaires de la ligne HF menant au récipient à traiter sont pratiquement négligeables. Le diamètre de la colonne ne dépasse pas celui d'un des récipients, par exemple d'une bouteille en PET (70mm).

Les adaptateurs LC sont reliés au générateur par un câble coaxial 81. Suivant les exigences techniques, on peut utiliser des adaptateurs LC à un ou deux contours.

Exemple 3.1

On a utilisé un générateur à haute fréquence 13,56 MHz dont la puissance moyenne est de 2,4kW et la puissance en impulsions est de 42kW. La puissance nécessaire pour la formation d'un film-barrière sur une bouteille PET de 0,5l est Pmoyenne = 0,4kW et Pimpulsion = 7kW. La quantité de décharge était 6. La quantité d'adaptateur LC était 6. On a utilisé un adaptateur à un contour avec autotransformation de l'inductance de sortie L. Le schéma de l'adaptateur est montré sur la Fig. 14a.

Les paramètres de l'adaptateur sont :

$$C = 250pF \; ; \; L = 0,5\mu H$$

Le réglage s'effectue en variant le point de contact avec l'enroulement L (ce réglage se fait une fois pour une charge R donnée (par exemple pour une bouteille).

Exemple 3.2

On a utilisé un générateur à haute fréquence 13,56 MHz dont la puissance moyenne était de 40kW et la puissance en impulsions 700kW. La puissance nécessaire pour la formation en film-barrière sur une bouteille PET de 0,5l est Pmoyenne = 0,4kW et Pimpulsion = 7kW. La quantité choisie de charges (dans ce cas de bouteilles PET (0,5l)) est 100. La quantité d'adaptateurs LC est de 100. On a utilisé un schéma d'adaptation à deux contours qui se caractérise, par rapport au schéma à un contour, par une influence plus faible de la charge sur le générateur. Le schéma de l'adaptateur LC est montré sur la Fig. 14b.

Les paramètres de l'adaptateur sont :

$$C_1 = 250pF \; ; \; L_1 = 0,5\mu H$$
$$C_2 = 85pF \; ; \; L_2 = 0.8\mu H$$

Le réglage suivant la charge R s'effectue en variant les inductances $L_1$ et $L_2$. Les capacités $C_1$ et $C_2$ sont constantes et ont des dimensions plus petites que les capacités variables de vide.

Un tel générateur avec 100 adaptateurs pour 100 charges peut être utilisé pour le traitement simultané de 100 bouteilles PET.

Exemple 3.3

On a utilisé un schéma de génération d'impulsions à haute fréquence pour l'alimentation de la décharge superficielle à filaments ramifiés. L'alimentation vient d'un générateur à haute fréquence par l'intermédiaire d'un adaptateur, et se diffère des schémas existant par le fait que le schéma utilise un système de circuit parallèle de résonance Fig. 14c.

Le condensateur $C_1$ (=100pF) est un condensateur de séparation $C_2$ (=30-100pF) est un condensateur d'ajustement, L(=0,5$\mu$H) est l'inductance utilisée. Ce shéma permet une adaptation en une étape. Les pertes sont réduites par rapport aux systèmes existants où elles peuvent être de 4 à 5 fois supérieures à l'énergie utile de la décharge.

Exemple 3.4

On a utilisé un schéma de génération d'impulsions à haute fréquence utilisant un générateur classique à haute fréquence sans adaptateur, en lui imposant un régime d'excitation indépendante. Les schémas d'autotransformation et de transformation sont effectués comme le montrent les figures 14d, et 14e.

Ces solutions ont l'avantage de l'exclusion des pertes dans l'adaptateur et permettent de brancher une multitude de charges identiques. Dans l'exemple présent on a mis en parallèle 4 décharges en impulsions dont la puissance moyenne était de 0,3kW. Le coefficient de transformation était de 1,1-1,3.

**Revendications**

1. Dispositif pour le traitement de surface de récipients (3) par plasma comprenant un système cinématique (2) pour le transport des récipient, et une pluralité de générateurs de plasma (4) travaillant à pression atmosphérique, chaque générateur étant destiné à traiter un récipient à la fois et chaque générateur comprenant un système d'atimentation de gaz de traitement (34), un système d'alimentation en courant électrique comprenant un bloc de distribution de courant électrique (30) et au moins un transistor (31) agissant comme interrupteur, ou un adaptateur LC, agencé pour alimenter le courant en impulsions, et une unité de contrôle (36) montée dans le corps du générateur ; chaque générateur étant sous forme d'une colonne d'un diamètre ou d'une largeur proche ou légèrement supérieur au diamètre ou à la largeur du récipient, et apte à générer une décharge du type réseau de filaments.

**2.** Dispositif selon la revendication 1, **caractérisé en ce que** l'unité de contrôle est agencée pour contrôler l'amplitude des impulsions, le taux de croissance du front de croissance des impulsions, la fréquence des impulsions et la durée entre les impulsions de courant électrique.

**3.** Dispositif selon l'une des revendications précédentes, **caractérisé en ce que** les générateurs de plasma sont disposés l'un à côté de l'autre sur un carrousel du système cinématique.

**4.** Dispositif selon l'une des revendication 1 ou 2, **caractérisé en ce que** le système cinématique comprend une zone d'accumulation des récipients au-dessus duquel sont disposés une pluralité de générateurs pour le traitement groupé (en "batch") des récipients.

**5.** Dispositif selon l'une des revendications précédentes, **caractérisé en ce que** le système d'alimentation en gaz comprend un distributeur de gaz et **en ce que** la source de courant, le distributeur de gaz et l'unité de contrôle comprenant un microcontrôleur déterminent le programme du traitement par plasma de manière individuelle pour chaque récipient.

**6.** Dispositif selon la revendication 5, **caractérisé en ce que** la source de courant, le distributeur de gaz et le microcontrôleur sont exécutés dans une même enceinte ou en blocs au-dessus du récipient à traiter.

**7.** Dispositif selon la revendication 4, **caractérisé en ce qu'**il comprend un guide pivotant pour aiguiller le chargement des récipients dans la zone de traitement par plasma (20).

**8.** Dispositif selon les revendications précédentes, **caractérisé en ce que** la zone de traitement comprend des rangs pour l'accumulation de récipients en rangs, de sorte que le traitement des récipients s'y effectue par rangs, au fur et à mesure du remplissage de ces rangs en récipients.

**9.** Dispositif selon la revendication 5, **caractérisé en ce qu'**il comprend deux zones complémentaires compartimentées avant et après la zone de traitement (20), par l'intermédiaire desquelles les containers sont respectivement rangés consécutivement dans la zone de traitement, et déchargés de la zone de traitement.

**10.** Dispositif selon l'une des revendications 1-9, **caractérisé en ce que** la source de courant du dispositif comprend une source centrale de courant continu bipolaire à haute tension alimentant les interrupteurs-transistors à haute vitesse et haute tension individuels de chaque générateur de plasma.

**11.** Dispositif selon l'une des revendications 1-9, **caractérisé en ce que** la source de courant du dispositif comprend une source centrale de courant continu unipolaire à haute tension alimentant les générateurs munis de ponts comprenant deux interrupteurs-transistors à haute vitesse et haute tension, de manière à créer une décharge du type "réseau de filaments".

**12.** Dispositif selon l'une des revendications 1-9, **caractérisé en ce que** la source de courant du dispositif comprend une source centrale de courant continu à haute tension alimentant les générateurs de plasma munis de systèmes individuels de transistors de champ pourvus chacun d'un circuit amplitude-phase C-R, le signal étant modulé par ordinateur, chacun de ces systèmes individuels alimentant en électricité une décharge du type "réseau de filaments", provoquée sur la surface intérieure du récipient à traiter.

**13.** Dispositif selon l'une des revendications 10 à 12, **caractérisé en ce que** les éléments à haute puissance du circuit de génération des impulsions de courant électrique sont refroidis de manière à fonctionner en régime de transfert de chaleur non-stationnaire.

**14.** Dispositif selon l'une des revendications précédentes, **caractérisé en ce que** le système cinématique comprend des canaux de transport à air (62) pour le déplacement des récipients par air soufflé dans le dispositif, les canaux de transport à air étant amovibles dans une zone de traitement par plasma (20) du dispositif afin de permettre l'accès par des électrodes (54a) des générateurs aux récipients.

**15.** Dispositif selon la revendication 2, **caractérisé en ce que** l'unité de contrôle gère un programme de distribution de portions de gaz pour former le mélange gazeux constituant le gaz de traitement utilisé lors du traitement par plasma des récipients.

## Claims

1. A device for treating the surface of containers (3) with a plasma, comprising a cinematic (2) system for the transport of the containers and a plurality of plasma generators (4) operating at atmospheric pressure, each generator adapted to treat one container at a time, and each generator comprising a treatment gas supply system (34), an electrical power supply system comprising a current distribution bloc (30) and at least one transistor (31) functioning as an interrupter, or an LC adapter, adapted for supplying current in pulses and a control unit (36) fixed inside the generator; each generator being in the form of a column of a diameter or a width close to or slightly superior to the diameter or the width of the container and adapted to generate a discharge in the form of a "network of filaments".

2. A device according to claim 1, **characterized in that** the control unit is adapted to control the amplitude of the pulses of electric current, the slope of the leading edge of these pulses, their frequency and the time elapsed between two successive pulses.

3. A device according to one of the preceding claims, **characterized in that** the plasma generators are placed side by side on a carrousel of the cinematic system.

4. A device according to one of claims 1 or 2, **characterized in that** the cinematic system comprises an accumulation zone for the containers and **in that** a plurality of generators are positioned above this system for a batch treatment of containers.

5. A device according to one of the preceding claims, **characterized in that** the gas supply system comprises a gas distributor and **in that** the current source, the gas distributor and the control unit comprising a microcontroller determine the plasma treatment program of each container individually.

6. A device according to claim 5, **characterized in that** the current source, the gas distributor and the microcontroller are provided in the same housing or as blocs above the container to be treated.

7. A device according to claim 4, **characterized in that** it comprises a pivoting guide for directing the loading of the containers in the plasma treatment zone (20).

8. A device according to the preceding claims, **characterized in that** the treatment zone comprises rows for storing rows of containers in such a manner that the treatment of the containers is carried out therein row by row, as and when the rows are filled by the containers.

9. A device according to claim 5, **characterized in that** it comprises two compartmented complementary zones upstream and downstream of the treatment zone (20), which are used for, respectively, placing the containers in row in the treatment zone and discharging the containers from the treatment zone.

10. A device according to one of claims 1 to 9, **characterized in that** the current source of the device comprises a central high voltage bipolar direct current source supplying the individual high speed and high voltage interrupter transistors of each plasma generator.

11. A device according to one of claims 1 to 9, **characterized in that** the current source of the device comprises a central high voltage unipolar direct current source supplying the generators provided with bridges comprising two high speed and high voltage interrupter transistors, in such a manner as to create a discharge in the form of a "network of filaments".

12. A device according to one of claims 1 to 9, **characterized in that** the current source of the device comprises a central high voltage direct current source supplying the plasma generators provided with individual field transistor systems, each having an CR amplitude-phase circuit, with the signal being modulated by a computer, each of these individual systems supplying electricity for a discharge in the form of a "network of filaments" on the inner surface of the container to be treated.

13. A device according to one of claims 10 to 12, **characterized in that** the high power elements of the circuit generating pulses of electric current are cooled in such a manner as to function in a non-steady heat transfer state.

14. A device according to one of the preceding claims, **characterized in that** the cinematic system comprises pneumatic transport channels (62) in which the containers are moved by an air stream, the pneumatic transport channels being movable in a plasma treatment zone (20) of the device, in order to enable the access of the generator electrodes (54a) to the containers.

15. A device according to claim 2, **characterized in that** the control unit controls the execution of a program of distribution of gas portions to form the gaseous mixture constituting the treatment gas used in the plasma treatment of the containers.

## Patentansprüche

1. Vorrichtung (3) zur Plasmabearbeitung der Oberfläche von Behältern mit einem kinematischen System (2) für den Transport der Behälter und einer Mehrzahl von bei Atmosphärendruck arbeitenden Plasmageneratoren (4), wobei jeder Generator dafür bestimmt ist, auf einmal jeweils einen Behälter zu behandeln, und jeder Generator ein System (34) für die Zufuhr von Behandlungsgas, ein System für die Zuführung von elektrischem Strom mit einem Stromverteilerblock (30) und zumindest einem als Unterbrecher wirkenden Transistor (31) oder einem LC-Adapter, der für die Zuführung des Stromes in Gestalt von Pulsen eingerichtet ist, sowie eine in den Generatorkörper eingebaute Steuereinheit (36) umfasst; und jeder Generator in Gestalt einer Säule mit einem Durchmesser oder einer Breite vorliegt, die Werte nahe bei oder leicht über dem Durchmesser oder der Breite des Behälters haben, und in der Lage ist, eine Entladung vom Typ eines Netzes von Filamenten zu erzeugen.

2. Vorrichtung nach Anspruch 1, **dadurch gekennzeichnet, dass** die Steuereinheit dafür eingerichtet ist, die Amplitude der Strompulse, die Steilheit der Pulsanstiegsflanke, die Pulsfrequenz und die Länge der Zeit zwischen elektrischen Strompulsen zu steuern.

3. Vorrichtung nach einem der vorangehenden Ansprüche, **dadurch gekennzeichnet, dass** die Plasmageneratoren nebeneinander auf einem Karussell des kinematischen Systems angeordnet sind.

4. Vorrichtung nach einem der Ansprüche 1 oder 2, **dadurch gekennzeichnet, dass** das kinematische System einen Sammelbereich für die Behälter umfasst, über dem eine Mehrzahl von Generatoren angeordnet ist, um die Behälter batchweise zu behandeln.

5. Vorrichtung nach einem der vorangehenden Ansprüche, **dadurch gekennzeichnet, dass** das Gaszufuhrsystem einen Gasverteiler umfasst, und **dadurch**, dass die Stromquelle, der Gasverteiler und die Steuereinheit einen Mikrocontroller umfassen, der das Plasmabehandlungsprogramm für jeden Behälter einzeln festlegt.

6. Vorrichtung nach Anspruch 5, **dadurch gekennzeichnet, dass** die Stromquelle, der Gasverteiler und der Mikrocontroller in ein und demselben Raum oder blockweise über dem zu behandelnden Behälter ausgelegt sind.

7. Vorrichtung nach Anspruch 4, **dadurch gekennzeichnet, dass** sie eine sich drehende Führung umfasst, um das Laden der Behälter in den Plasmabehandlungsbereich (20) zu lenken.

8. Vorrichtung nach den vorangehenden Ansprüchen, **dadurch gekennzeichnet, dass** der Behandlungsbereich Reihen für die reihenweise Sammlung von Behältern umfasst, damit die Behandlung der Behälter reihenweise gemäss der Füllung dieser Reihen mit Behältern erfolgt.

9. Vorrichtung nach Anspruch 5, **dadurch gekennzeichnet, dass** sie vor und hinter dem Behandlungsbereich (20) zwei komplementäre, unterteilte Bereiche umfasst, durch die die Behälter aufeinanderfolgend im Behandlungsbereich aufgereiht bzw. aus dem Behandlungsbereich entlassen werden.

10. Vorrichtung nach einem der Ansprüche 1 bis 9, **dadurch gekennzeichnet, dass** die Stromquelle der Vorrichtung eine zentrale bipolare Hochspannungs-Gleichstromquelle umfasst, die die einzelnen Hochgeschwindigkeits-Hochspannungs-Schalttransistoren jedes Plasmagenerators speist.

11. Vorrichtung nach einem der Ansprüche 1 bis 9, **dadurch gekennzeichnet, dass** die Stromquelle der Vorrichtung eine zentrale unipolare Hochspannungs-Gleichstromquelle umfasst, die die Generatoren, die mit Brükken aus je zwei Hochgeschwindigkeits-Hochspannungs-Schaltltransistoren versehen sind, so speist, dass eine Entladung vom Typ eines Netzwerks von Filamenten erzeugt wird.

12. Vorrichtung nach einem der Ansprüche 1 bis 9, **dadurch gekennzeichnet, dass** die Stromquelle der Vorrichtung eine zentrale Hochspannungs-Gleichstromquelle umfasst, die die Plasmageneratoren speist, die mit einzelnen Systemen von Feldeffekttransistoren versehen sind, jedes mit einer R-C-Amplituden-Phasen-Schaltung versehen, wobei das Signal durch Rechner moduliert wird und jedes dieser einzelnen Systeme eine Entladung vom Typ eines Netzwerkes von Filamenten, die auf der inneren Oberfläche des zu behandelnden Behälters hervorgerufen wird, mit Strom versorgt.

13. Vorrichtung nach einem der Ansprüche 10 bis 12, **dadurch gekennzeichnet, dass** die Hochleistungselemente der Schaltung für die Erzeugung der elektrischen Strompulse so gekühlt werden, dass sie unter Bedingungen einer nichtstationären Wärmeübertragung funktionieren.

14. Vorrichtung nach einem der vorangehenden Ansprüche, **dadurch gekennzeichnet, dass** das kinematische System pneumatische Kanäle (62) für eine Fortbewegung der Behälter durch in die Vorrichtung eingeblasene Luft umfasst, wobei die pneumatischen Kanäle abnehmbar in einem Plasmabehandlungsbereich (20) der Vorrichtung liegen, um den Zugriff von Elektroden (54a) der Generatoren auf die Behälter zu ermöglichen.

**15.** Vorrichtung nach Anspruch 2, **dadurch gekennzeichnet, dass** die Steuereinheit ein Programm für die Verteilung von Gasanteilen handhabt, um das Gasgemisch zu bilden, aus dem das Behandlungsgas besteht, das bei der Plasmabehandlung der Behälter verwendet wird.

**FIG. 1**

**FIG. 2**

EP 1 497 846 B1

FIG.3

FIG.4

FIG.5a

FIG.5b

FIG.6

FIG. 7a

EP 1 497 846 B1

FIG.7b

FIG.8

GAZ

RESEAU
ELECTRIQUE

GAZ

FIG. 9

FIG. 10

**FIG. 11**

FIG. 12

FIG. 13

FIG.14 a

FIG.14 b

FIG.14 c

FIG.14 d

FIG.14 e

**EP 1 497 846 B1**

## RÉFÉRENCES CITÉES DANS LA DESCRIPTION

**Documents brevets cités dans la description**

- WO IB0201001 A
- WO 02076511 A2
- WO 9946964 A

**Littérature non-brevet citée dans la description**

- **SIDEL.** *SIDEL News,* Septembre 2001, 8, 9
- **TETRAPAK.** *Tetrapak Business Area Plastics,* Septembre 2001
- **KRONES.** *Krones News,* Septembre 2001